# EUROPEAN PATENT SPECIFICATION

(11) **EP 4 316 408 B1**
(45) Date of publication and mention of the grant of the patent: **03.12.2025**
(21) Application number: 22794334.7
(22) Date of filing: 03.03.2022
(51) Int. Cl.: A61B 34/37

(54) **INPUT DEVICE AND SURGICAL ROBOT**
EINGABEVORRICHTUNG UND CHIRURGISCHER ROBOTER
DISPOSITIF D'ENTRÉE ET ROBOT CHIRURGICAL

(30) Priority: 28.04.2021 CN 202110469703
(43) Date of publication of application: 07.02.2024
(73) Proprietor: Cornerstone Technology (Shenzhen) Limited, Shenzhen, Guandong 518066 (CN)
(72) Inventor: SUN, Rongjian, Shenzhen, Guangdong 518066 (CN); LU, Yueqiang, Shenzhen, Guangdong 518066 (CN); XIE, Tian, Shenzhen, Guangdong 518066 (CN)
(74) Representative: Dennemeyer & Associates S.A.
(86) International application number: PCT/CN2022/078979
(87) International publication number: WO 2022/227855

(56) References cited:
- EP-A1- 4 331 523
- WO-A1-2020/041513
- WO-A1-2020/041513
- WO-A2-2018/112227
- CN-A- 111 265 303
- CN-A- 111 885 979
- CN-A- 113 180 836
- CN-A- 113 197 673
- KR-A- 20140 078 150
- US-A1- 2003 060 927
- US-A1- 2003 060 927
- US-A1- 2008 046 122
- US-A1- 2018 256 266

## Description

### TECHNICAL FIELD

The various embodiments described in this document relate to the technical field of medical instruments, and in particular to an input device and a surgical robot.

### BACKGROUND

Most medical surgical robots adopt a master-slave control architecture, where a doctor operates a master manipulator of an input device to control a movement of a slave manipulator such as an end effector through remote communication and a computer. For example, the doctor controls the end effector, such as a pair of forceps, a pair of scissors, a clamp, etc., by opening and closing a grip handle of the input device.

For a measurement of a rotation angle of a rotating unit of a known input device, the rotation of the rotating unit is usually transmitted to a bevel gear, and then an angle of the bevel gear is measured by an encoder. Due to a gap between the bevel gears, there is a certain difference between the angle measured by the encoder and an actual rotation angle of the rotating unit, and the angle measured may be inaccurate.

In addition, an included angle between members of a grip mechanism of the input device is an important input parameter for control, so it is extremely important for accuracy and stability of the measurement of the included angle. In the known angle detection method of the grip mechanism, a Hall sensing chip is usually arranged in a certain part of the input device, and a magnetic element is arranged in another part of the input device (for example, a rotation support is provided with the Hall sensing chip, and the grip mechanism is provided with the magnetic element), and the part with the magnetic element moves to change the magnetic field intensity around the Hall sensing chip. However, the change in magnetic field intensity is not linear due to a non-linear variation of a distance between the magnetic element and the Hall sensing chip, thus the included angle cannot be detected directly, and additional sensors are required for angle calibration.
Reference document WO2020041513A1 discloses a control input device including a base member and a roll member rotatable about a central axis with respect to the base member in a roll degree of freedom. A switch contact portion is rotatable with the roll member and moveable to multiple positions in a switch degree of freedom. A first sensor element is coupled to and moveable with the switch contact portion and can be a passive element. A base sensor element is coupled to the base member and configured to sense a proximity of the first sensor element to the base sensor element, and to output a signal indicative of a current position of the switch contact portion in the switch degree of freedom independently of a rotational orientation of the roll member in the roll degree of freedom.
Reference document US20030060927A1 discloses an input device for robotic surgery mechanically transmits a grip signal across a first joint coupling a handle to a linkage supporting the handle. The handle is removable and replaceable, allows unlimited rotation about the joint, and may optionally include a touch sensor to inhibit movement of a surgical end effector when the hand of the surgeon is not in contact with the handle.
Reference document WO2018112227A2 discloses a controller including a central member, a grip member coupled to the central member and moveable in a grip degree of freedom, a shaft coupled to the grip member, and an actuator coupled to the shaft and operative to output an actuator force on the shaft. The actuator force causes a grip force to be applied via the shaft to the grip member in the grip degree of freedom. Reference document KR 2014 0078150 A discloses an input device in which opening and closing of the gripping portion is sensed by detecting a direction of a rotating magnetic element.

### SUMMARY

The present invention is set out in the appended claims.

### BRIEF DESCRIPTION OF THE DRAWINGS

The following drawings of this application are incorporated herein as a part of this application to understand this application. Embodiments of the present disclosure and their descriptions are shown in the drawings to explain the principles of the present disclosure.

In the drawings:
FIG. 1 is a schematic perspective view of an input device of a surgical robot according to some embodiments of the present disclosure, where a first grip member and a second grip member are opened to a maximum included angle;
FIG. 2 is an exploded perspective view of the input device in FIG. 1;
FIG. 3 is another exploded perspective view of the input device in FIG. 1;
FIG. 4 is a schematic sectional view of the input device in FIG. 1, where the first grip member and the second grip member are opened to the maximum included angle;
FIG. 5 is another schematic sectional view of the input device in FIG. 1, where the first grip member and the second grip member are closed to a minimum included angle; and
FIG. 6 is a schematic diagram of a partial structure of a mounting base of the input device in FIG. 2, showing an induction board, a first detection component, and second detection components.

### DETAILED DESCRIPTION OF THE EMBODIMENTS

In the following description, a large number of specific details are set forth to provide a more thorough understanding of this application. However, it is apparent to those skilled in the art that the embodiments of the present disclosure can be implemented without the need for one or more of these details. In other examples, in order to avoid confusion with the embodiments of the present disclosure, some well-known technical features in the art have not been described.

In order to thoroughly understand the embodiments of the present disclosure, a detailed structure is set forth in the follow description. Obviously, the implementation of the embodiment of the present disclosure is not limited to the specific details familiar to those skilled in the art. It should be noted that the ordinal numbers such as "first" and "second" quoted in this application are only signs, and do not have any other meaning, such as a specific order. Moreover, for example, the term "first component" itself does not imply the existence of "second component", and the term "second component" itself does not imply the existence of "first component". The terms "upper", "lower", "front", "rear", "left" and "right" and similar expressions used in this application are for illustration purposes only, and are not for limitation.

An input device 100 and a surgical robot are provided according to some embodiments of the present disclosure. The surgical robot includes an input device 100, an end effector may be controlled by a doctor operating the input device 100 via remote communication and a computer, and the end effector may be a pair of forceps, a pair of scissors, a clamp, etc. Other structures of the surgical robot may be designed according to the existing technology in the field, and are not described in detail herein.

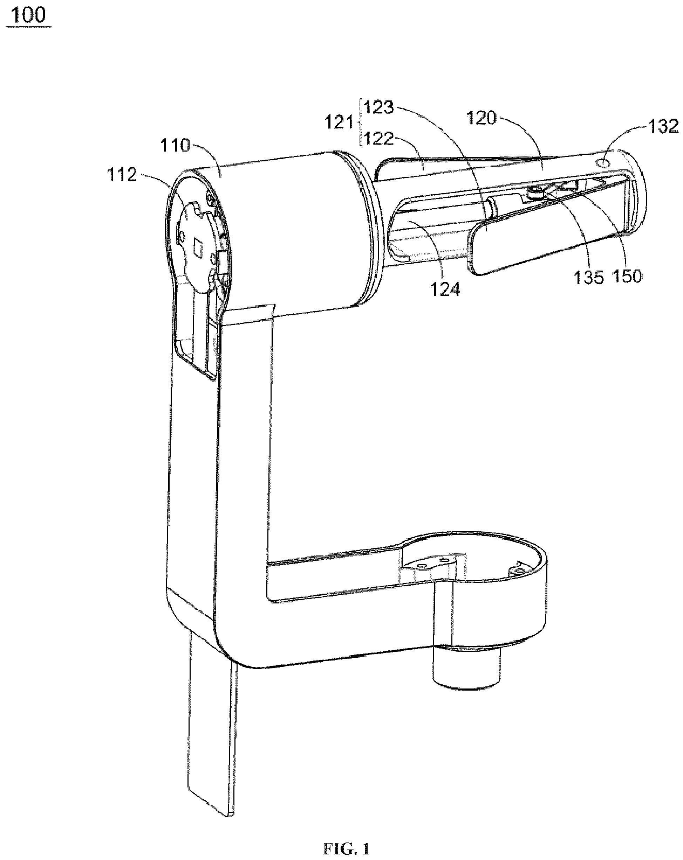

The input device 100 according to some embodiments of the present disclosure is described in detail with reference to FIG. 1 to FIG. 6.

As shown in FIG. 1 to FIG. 6, the input device 100 mainly includes a mounting base 110, a rotation support 120, a grip mechanism 121, a connection member 124, a magnetic element 140, at least one first detection component 114, at least one second detection component 115, an induction board 112, and a signal processing board 113.

The mounting base 110 is configured to fix the input device 100 on a desktop or other structure, and to mount a driving motor (not shown). The rotation support 120 is disposed on the mounting base 110 and rotatable around a first central axis relative to the mounting base 110. For example, the first central axis may be a central axis of the rotation support 120.

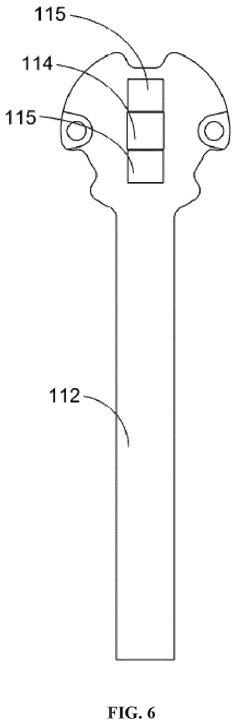

The grip mechanism 121 is disposed on the rotation support 120, and is capable of being elastically opened and closed relative to the rotation support 120. The connection member 124 is connected with the grip mechanism 121 and movable in a direction substantially parallel to the first central axis with the opening and closing of the grip mechanism 121. As shown in FIG. 6, the induction board 112 and the signal processing board 113 are mounted in the mounting base 110. The first detection component 114 and the second detection component 115 are mounted on the induction board 112. The signal processing board 113 is configured to receive and process information data transmitted by the induction board 112 (for example, the data detected by the first detection component 114 and the second detection component 115).

The first detection component 114 is configured to detect a magnetic field direction of the magnetic element. For example, the rotation angle of the rotation support 12 may be obtained based on a change of the rotation of the magnetic field with the magnetic element 140 detected by the first detecting component 114. The second detection component 115 is configured to detect the magnetic field intensity of the magnetic element 140. For example, the included angle between members of the grip mechanism 121 may be obtained based on a change in distance from the magnetic element 140 detected by the second detection component 115. Specifically, the magnetic element 140 may be a magnet. In some embodiments, the magnetic element may also be an alloy with magnetism. In some embodiments, the first detection component 114 and the magnetic element 140 are disposed in a straight line substantially parallel to the first central axis.

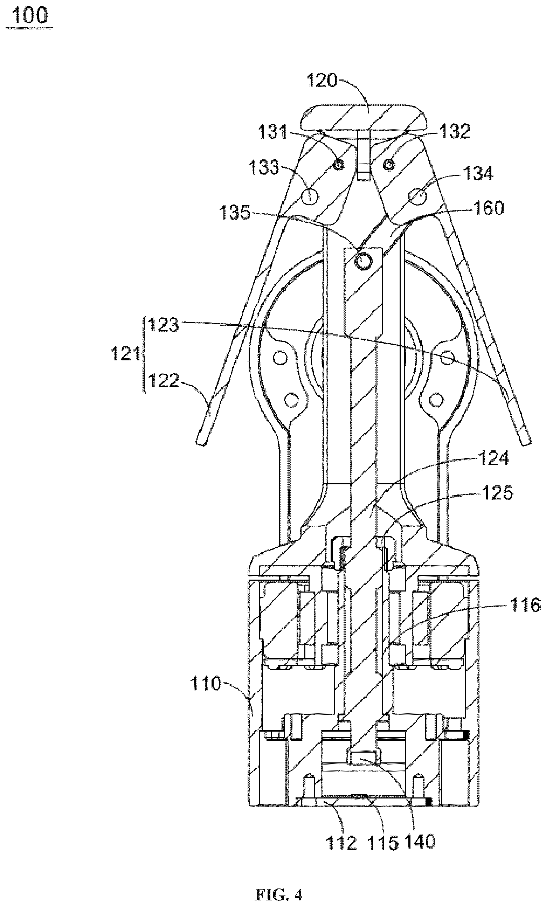

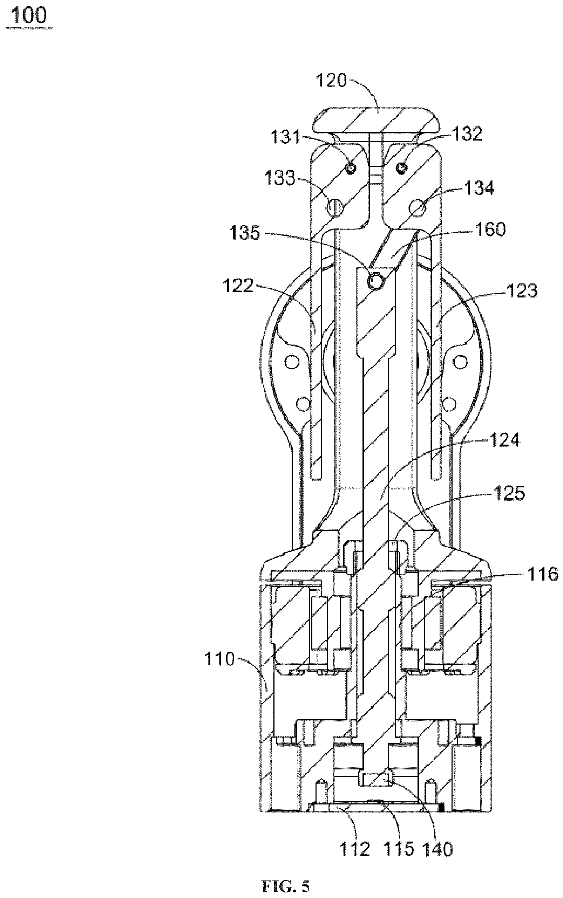

In this embodiment, as shown in FIG. 4 and FIG. 5, the magnetic element 140 is disposed on the connection member 124. As the grip mechanism 121 is being opened or closed, the connection member 124 moves together with the connection member 124 in a direction substantially parallel to the first central axis. In this way, the distance between the magnetic element 140 and the second detection component 115 is changed, and the included angle between members of the grip mechanism 121 is obtained by detection of the change. Furthermore, the magnetic element 140 may also rotate with the rotation support 120 to change the relative rotation angle between the magnetic element 140 and the first detection component 114, so that the first detection component 114 may obtain the rotation angle of the grip mechanism 121 by detection of a rotation angle of the magnetic field of the magnetic element 140. In this embodiment, the included angle and the rotation angle of the grip mechanism 121 can be detected using only one magnetic element 140, thus reducing the number of detection components (such as sensor components). At the same time, the first detection component 114 and the second detection component 115 are disposed on the same signal processing board 113, which reduces the number of line connections, and the two detection components can be placed in the same coding system for processing.

As shown in FIG. 1 to FIG. 5, the grip mechanism 121 includes a first grip member 122 and a second grip member 123, the first grip member 122 and the second grip member 123 are pivotally connected to the rotation support 120, and the first grip member 122 and the second grip member 123 are cooperatively movable through a pair of spur gears, so that the first grip member 122 and the second grip member 123 pivot synchronously. One end of the first grip member 122 is pivotally disposed at one side of the rotation support 120. One end of the second grip member 123 is pivotally disposed on another side of the rotation support 120 opposite to the first grip member 122. The first grip member 122 and the second grip member 123 may transition (e.g., rotate) within the range of maximum included angle and minimum included angle. When the two grip members move closest to the rotation support 122, that is, the grip mechanism 121 is at a close position, the included angle between the two grip members is minimum, and when the two grip members move farthest from the rotation support 122, that is, the grip mechanism 121 is at an open position, the included angle between the two grip members is maximum. In this embodiment, the first grip member 122 and the second grip member 123 are symmetrically disposed about the first central axis. Those skilled in the art should understand that the installation positions of the first grip member 122 and the second grip member 123 are not limited to this embodiment.

In some embodiments, the magnetic element may be directly disposed on the rotation support or indirectly disposed on the rotation support through an intermediate connection member (not shown), so that the magnetic element can rotate with the rotation of the rotation support, the rotation angle of the magnetic element can be changed and detected by the first detection component to obtain the rotation angle of the rotation support.

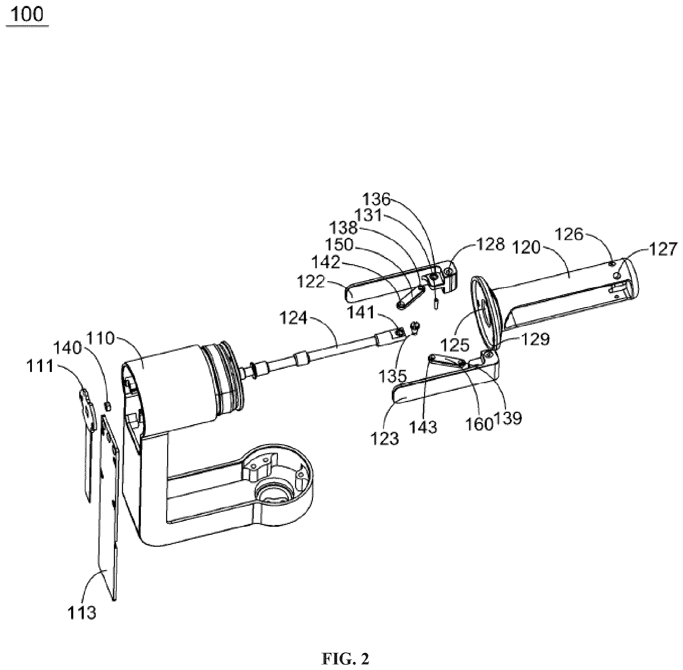

In this embodiment, one end of the connection member 124 is pivotally connected with the first grip member 122 and the second grip member 123, and the connection member 124 is rotatable around its central axis. As shown in FIG. 2 to FIG. 4, the magnetic element 140 is disposed at one end of the connection member 124 opposite to the end to which the first grip member 122 and the second grip member 123 are connected. The magnetic element 140 is spaced apart from the first detection component 114 and the second detection component 115 by a distance in a direction substantially parallel to the first central axis, so as to prevent the magnetic element 140 from interfering with the first detection component 114 and the second detection component 115 when the connection member 124 moves in a direction substantially parallel to the first central axis, and to avoid the magnetic element 140 being too close to the first detection component 114 and the second detection component 115, which may affect the detection of the first detection component 114 and the second detection component 115 (for example, affecting the detection accuracy).

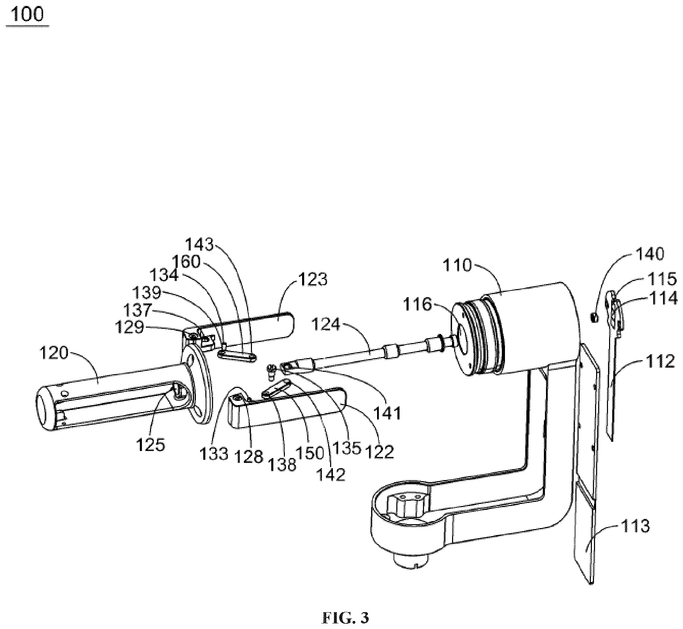

As shown in FIG. 3 and FIG. 6, in this embodiment, the input device 100 includes a first detection component 114 and two second detection components 115. In an embodiment, in the direction along the first central axis, the position of the first detection component 114 and the position of the magnetic element 140 are aligned. The first detection component 114 and the two second detection components 115 are disposed on a plane substantially perpendicular to the first central axis. The two second detection components 115 are disposed in a vertical array with the first detection component 114 located therebetween. The first detection component 114 and the second detection component 115 may be Hall sensing chips. It should be understood that the position relationship between the first detection component 114 and the second detection component 115 is not limited to this embodiment. As needed, the two second detection components 115 may be disposed in a horizontal array with the first detection component 114 located therebetween, or may be disposed at any other suitable position.

During detection, the magnetic field intensity detected by the second detection component 115 does not change in a linear relation with the change of the distance between the second detection component and the magnetic element, but the relation of the changes may be approximately regarded as linear within a certain range. In this embodiment, the detected magnetic field intensity within this range is taken as an effective basis to calculate or match the included angles between the first grip member 122 and the second grip member 123 at this magnetic field intensity. In addition, the first detection component 114 and the second detection component 115 are combined in a set of encoder system, which can simplify the detection scheme, simplify the structure of the input device 100, reduce the difficulty of wiring, and increase the reliability of the entire input device 100.

Guide holes are defined on the mounting base 110 and/or the rotation support 120, and extend in a direction substantially parallel to the first central axis. The connection member 124 may movably pass through the guide hole, for example, the connection member 124 may move along the extension direction of the guide hole. The connection member 124 may be configured as a shaft, a rod, an elongated shape or any suitable shape. As shown in FIG. 2 to FIG. 5, the guide holes include a first guide hole 116 defined by the mounting base 110 and a second guide hole 125 defined by the rotation support 120. The first guide hole 116 and the second guide hole 125 are both constructed as circular through holes, and the first guide hole 116 is coaxial with the second guide hole 125. In an embodiment, the central axes of the first guide hole 116 and the second guide hole 125 coincide with the first central axis.

The input device 100 further includes an elastic element (not shown), and opposite ends of the elastic element are connected to the first grip member 122 and the second grip member 123, respectively. The elastic element applies pushing forces to the first grip member 122 and the second grip member 123 that are opened or tended to be opened with the pushing force, so that the first grip member 122 and the second grip member 123 can be kept at the maximum opening position, that is, the maximum included angle, without external force. The elastic element may be a compression spring. In some embodiments, the elastic element may be elastic sheet or other elastically deformable connection members.

As shown in FIG. 1 to FIG. 5, the input device 100 further includes a first pin 131 and a second pin 132 substantially parallel to the first pin 131, and the first pin 131 is substantially perpendicular to the first central axis. One end of the first grip member 122 is pivotally connected to the rotation support 120 through the first pin 131, one end of the second grip member 123 is rotatably connected to the rotating shaft 120 through the second pin 132.

Specifically, a first mounting hole 126 and a second mounting hole 127 are defined by the rotating support 120, a first connecting hole 128 is correspondingly defined by the first grip member 122, and a second connecting hole 129 is correspondingly defined by the second grip member 123. Each of the first mounting hole 126, the second mounting hole 127, the first connecting hole 128 and the second connecting hole 129 may be configured as a circular through hole. The first pin 131 is inserted into the first mounting hole 126 and the first connecting hole 128 to pivotally connect the first grip member 122 to the rotation support 120. The second pin 132 is inserted into the second mounting hole 127 and the second connecting hole 129 to pivotally connect the second grip member 123 to the rotation support 120.

As shown in FIG. 3 to FIG. 5, the input device 100 further includes a first connecting rod 150, a second connecting rod 160, a third pin 133, a fourth pin 134 and a fifth pin 135. The third pin 133, the fourth pin 134 and the fifth pin 135 are substantially parallel to each other and substantially perpendicular to the first central axis. One end of the first connecting rod 150 is pivotally connected to the first grip member 122 through the third pin 133, and one end of the second connecting rod 160 is pivotally connected to the second grip member 123 through the fourth pin 134, the other end of the first connecting rod 150 and the other end of the second connecting rod 160 are pivotally connected to one end of the connection member 124 near the first grip member 122 and the second grip member 123 through the fifth pin 135.

Specifically, a third mounting hole 136 is defined by the first grip member 122, and a third connecting hole 138 is defined at one end of the first connecting rod 150. The third pin 133 is inserted into the third mounting hole 136 and the third connecting hole 138 to pivotally connect the first connecting rod 150 to the first grip member 122. A fourth mounting hole 137 is defined by the second grip member 123, and a fourth connecting hole 139 is defined at one end of the second connecting rod 160. The fourth pin 134 is inserted into the fourth mounting hole 137 and the fourth connecting hole 139 to pivotally connect the second connecting rod 160 to the second grip member 123. Each of the third mounting hole 136, the third connecting hole 138, the fourth mounting hole 137, and the fourth connecting hole 139 may be configured as a circular through hole.

A fifth mounting hole 141 is defined at one end of the connection member 124, a fifth connecting hole 142 is correspondingly defined at the other end of the first connecting rod 150, and a sixth connecting hole 143 is correspondingly defined at the other end of the second connecting rod 160. The fifth pin 135 is inserted into the fifth mounting hole 141, the fifth connecting hole 142 and the sixth connecting hole 143 to pivotally connect the other end of the first connecting rod 150 (i.e., the end farther away from the first grip member 122) and the other end of the second connecting rod 160 (i.e., the end farther away from the second grip member 123) to the connection member 124. Each of the fifth mounting hole 141, the fifth connecting hole 142 and the sixth connecting hole 143 may be configured as a circular through hole.

The working principle of the input device 100 according to the embodiments of the present disclosure is described in detail below.

As shown in FIG. 4, when the first grip member 122 and the second grip member 123 of the input device 100 are opened to the maximum included angle, that is, the grip mechanism 121 is at the open position, the magnetic element 140 is farthest from the first detection component 114 and the second detection component 115. When forces are exerted (by, e.g., fingers) on the first grip member 122 and the second grip member 123 so that the two grip members transition (e.g. are closed) toward the minimum included angle, the first grip member 122 and the second grip member 123 exert forces on the first connecting rod 150 and the second connecting rod 160, respectively. In this way, the forces are transmitted to the connection member 124, which drive the connection member 124 to move linearly along its axial direction. As shown in FIG. 5, when the first grip member 122 and the second grip member 123 are closed to the minimum included angle, that is, the grip mechanism 121 is at the close position, the magnetic element 140 is closest to the first detection component 114 and the second detection component 115. During pivot of the first grip member 122 and the second grip member 123, the second detection component 115 can detect the magnetic field intensity of the magnetic element 140 at different positions, the data information detected by the second detection component 115 is transmitted to the signal processing board 113, and then processed by the signal processing board 113 to obtain the information about the included angle of the first grip member 122 and the second grip member 123.

When the rotation support 120 rotates, the first grip member 122, the second grip member 123, the connection member 124 and the magnetic element 140 rotate synchronously with the rotation support 120, and there are components of the magnetic field in both x and y directions in the plane where the first detection component 114 is located. The data information detected by the first detection component 114 is transmitted to the signal processing board 113 which calculates the current angle of the magnetic element 140 relative to the induction board 112 through data calculation. This angle reflects the current rotation angle of the rotation support 120. During the rotation of the rotation support 120, the included angles between the first grip member 122 and the second grip member 123 may change to move the connection member 124 and thus the magnetic element 140, resulting in a change in the position or distance of the magnetic element 140 relative to the first detection component 114. However, for each specific rotation angle of the rotation support 120, the calculation results of the components of the magnetic field in the x and y directions located in the plane where the first detection component 114 is located (for example, the directions of the components of the magnetic field) do not change with the change of the distance between the first detection component 114 and the magnetic element 140. Therefore, regardless of the included angles between the first grip member 122 and the second grip member 123, the rotation angle of the rotation support 120 relative to the mounting base 110 can be calculated and detected by the first detection component 114 and the signal processing board 113.

Unless otherwise defined, technical and scientific terms used herein have the same meaning as commonly understood by those skilled in the technical field of this application. The terminology used herein is only for describing specific implementation purposes and is not intended to limit the application. The terms such as "dispose" appearing in this specification can refer to either a component directly attached to another component or a component attached to another component through middleware. A feature described in one embodiment herein can be applied to another embodiment alone or in combination with other features, unless the feature is not applicable in the other embodiment or otherwise specified.

The application has been described by the above embodiments, but it should be understood that the above embodiments are only used for the purpose of illustration and description, and are not intended to limit the application to the described embodiments. It should be understood by those skilled in the art that many more variations and modifications can be made according to the teaching of this application, and these variations and modifications are all within the scope of protection claimed in this application.

## Claims

1. An input device (100), comprising:
a mounting base (110);
a rotation support (120), rotatable relative to the mounting base (110) about a first central axis;
a grip mechanism (121), disposed on the rotation support (120) and transitionable between an open position and a close position;
a connection member (124), connected with the grip mechanism (121) and movable in a direction parallel to the first central axis with opening and closing of the grip mechanism (121), the connection member being rotatable about the first central axis with rotation of the rotation support (120);
a magnetic element (140);at least one first detection component (114), disposed on the mounting base (110); and
at least one second detection component (115), disposed on the mounting base (110) and configured to detect a magnetic field intensity of the magnetic element (140); and
a signal processing board (113) mounted in the mounting base (110), the signal processing board (113) being configured to obtain an included angle between members of the grip mechanism (121) based on the magnetic field intensity of the magnetic element (140) detected by the at least one second detection component (115);
**characterized in that**,
the magnetic element (140) is disposed on the connection member (124), and the magnetic element (140) is movable and rotatable with the connection member (124); and
the at least one first detection component (114) is configured to detect components of a magnetic field in x and y directions in a plane where the at least one first detection component (114) is located; and the signal processing board (113) is further configured to obtain a rotation angle of the rotation support (120) based on the components of the magnetic field in x and y directions detected by the at least one first detection component (114).

2. The input device (100) according to claim 1, wherein the grip mechanism (121) comprises a first grip member (122) and a second grip member (123), which are respectively pivotally disposed on the rotation support (120).

3. The input device (100) according to claim 2, wherein the first grip member (122) and the second grip member (123) are disposed on two opposite sides of the rotation support (120) respectively, and the first grip member (122) and the second grip member (123) are cooperatively movable, and/or
the connection member (124) has one end to which the first grip member (122) and the second grip member (123) are pivotally connected, and one other end on which the magnetic element (140) is disposed and which is located away from the first grip member (122) and the second grip member (123).

4. The input device (100) according to claim 2 or claim 3, further comprising a first pin (131) and a second pin (132) parallel to the first pin (131), wherein the first grip member (122) has one end pivotally connected to the rotation support (120) through the first pin (131), and the second grip member (123) has one end pivotally connected to the rotation support (120) through the second pin (132).

5. The input device (100) according to claim 4, further comprising a first connecting rod (150) and a second connecting rod (160), wherein the first connecting rod (150) has two ends pivotally connected to the first grip member (122) and the connection member (124), respectively, and the second connecting rod (160) has two ends pivotally connected to the second grip member (123) and the connection member (124), respectively.

6. The input device (100) according to claim 5, further comprising a third pin (133), a fourth pin (134) and a fifth pin (135), which are parallel to each other, wherein one of the two ends of the first connecting rod (150) is pivotally connected to the first grip member (122) through the third pin (133), one of the two ends of the second connecting rod (160) is pivotally connected to the second grip member (123) through the fourth pin (134), and the other one of the two ends of the first connecting rod (150) and the other one of the two ends of the second connecting rod (160) are pivotally connected to the connection member (124) through the fifth pin (135).

7. The input device (100) according to claim 6, wherein
the third pin (133) is parallel to the first pin (131), and/or
a central axis of the third pin (133) is substantially perpendicular to the first central axis.

8. The input device (100) according to any one of claims 1 to 7, wherein
each of the at least one first detection component (114) is spaced apart from the magnetic element (140) in a direction parallel to the first central axis, and/or
the at least one first detection component (114) and the magnetic element (140) are disposed in a straight line parallel to the first central axis.

9. The input device (100) according to any one of claims 1 to 8, wherein the at least one first detection component (114) is aligned with the magnetic element (140) along the first central axis.

10. The input device (100) according to claim 9, wherein two second detection components (115) are provided,
wherein the two second detection components (115) are spaced apart from the magnetic element (140) in a direction parallel to the first central axis, and/or
wherein one first detection component (114) is provided, the first detection component (114) and the two second detection components (115) are disposed on a plane perpendicular to the first central axis, and the two second detection components (115) are disposed on two sides of the first detection component (114).

11. The input device (100) according to any one of claims 1 to 10, wherein one or both of the mounting base (110) and the rotation support (120) defines a guide hole, the guide hole extends in a direction parallel to the first central axis, and the connection member (124) is configured to movably pass through the guide hole.

12. The input device (100) according to claim 11, wherein the guide hole comprises a first guide hole (116) defined by the mounting base (110) and a second guide hole (125) defined by the rotation support (120), and the first guide hole (116) is coaxial with the second guide hole (125).

13. A surgical robot, comprising an input device (100) according to any one of claims 1 to 12.

## Patentansprüche

1. Eingabegerät (100), umfassend:
eine Montagebasis (110);
einen drehbaren Träger (120), der relativ zur Montagebasis (110) um eine erste Mittelachse drehbar ist;
einen Greifmechanismus (121), der auf dem drehbaren Träger (120) angeordnet ist und von einer offenen Position in eine geschlossene Position gebracht werden kann;
ein Verbindungselement (124), das mit dem Greifmechanismus (121) verbunden ist und sich beim Öffnen und Schließen des Greifmechanismus (121) in einer Richtung parallel zur ersten Mittelachse bewegen kann, wobei das Verbindungselement mit der Drehung des drehbaren Trägers (120) um die erste Mittelachse drehbar ist;
ein Magnetelement (140); mindestens eine erste Erfassungskomponente (114), die auf der Montagebasis (110) angeordnet ist; und
mindestens eine zweite Erfassungskomponente (115), die auf der Montagebasis (110) angeordnet und so konfiguriert ist, dass sie eine Magnetfeldstärke des Magnetelements (140) erfasst; und
eine Signalverarbeitungskarte (113), die in der Montagebasis (110) montiert ist, wobei die Signalverarbeitungskarte (113) so konfiguriert ist, dass sie einen Winkel zwischen den Elementen des Greifmechanismus (121) auf der Grundlage der von der mindestens einen zweiten Erfassungskomponente (115) erfassten Magnetfeldstärke des Magnetelements (140) ermittelt;
**dadurch gekennzeichnet, dass**
das Magnetelement (140) auf dem Verbindungselement (124) angeordnet ist und das Magnetelement (140) zusammen mit dem Verbindungselement (124) beweglich und drehbar ist; und
die mindestens eine erste Erfassungskomponente (114) so konfiguriert ist, dass sie die Komponenten eines Magnetfelds in x- und y-Richtung in einer Ebene erfasst, in der sich die mindestens eine erste Erfassungskomponente (114) befindet; und die Signalverarbeitungskarte (113) ist ferner so konfiguriert, dass sie einen Drehwinkel des drehbaren Trägers (120) auf der Grundlage der Magnetfeldkomponenten in x- und y-Richtung ermittelt, die von der mindestens einen ersten Erfassungskomponente (114) erfasst werden.

2. Eingabegerät (100) nach Anspruch 1, wobei der Greifmechanismus (121) ein erstes Greifelement (122) und ein zweites Greifelement (123) umfasst, die jeweils schwenkbar an dem drehbaren Träger (120) angeordnet sind.

3. Eingabegerät (100) nach Anspruch 2, wobei das erste Greifelement (122) und das zweite Greifelement (123) jeweils an zwei gegenüberliegenden Seiten des drehbaren Trägers (120) angeordnet sind, und das erste Greifelement (122) und das zweite Greifelement (123) zusammenwirkend beweglich sind, und/oder
das Verbindungselement (124) ein Ende aufweist, an dem das erste Greifelement (122) und das zweite Greifelement (123) schwenkbar verbunden sind, und ein weiteres Ende aufweist, an dem das Magnetelement (140) angeordnet ist und das von dem ersten Greifelement (122) und dem zweiten Greifelement (123) beabstandet ist.

4. Eingabegerät (100) gemäß Anspruch 2 oder Anspruch 3, die außerdem einen ersten Stift (131) und einen zweiten Stift (132) parallel zum ersten Stift (131) umfasst, wobei das erste Greifelement (122) ein Ende aufweist, das über die erste Spindel (131) schwenkbar mit der drehbaren Träger (120) verbunden ist, und das zweite Greifelement (123) ein Ende aufweist, das über die zweiten Stift (132) schwenkbar mit der drehbaren Halterung (120) verbunden ist zweiten Stift (132) mit dem drehbaren Träger (120) verbunden

5. Eingabegerät (100) gemäß Anspruch 4, die außerdem eine erste Verbindungsstange (150) und eine zweite Verbindungsstange (160) umfasst, wobei die erste Verbindungsstange (150) zwei Enden aufweist, die schwenkbar mit dem ersten Greifelement (122) bzw. dem Verbindungselement (124) verbunden sind, und die zweite Verbindungsstange (160) zwei Enden aufweist, die schwenkbar mit dem zweiten Greifelement (123) bzw. dem Verbindungselement (124) verbunden sind.

6. Eingabegerät (100) gemäß Anspruch 5, die außerdem einen dritten Stift (133), einen vierten Stift (134) und einen fünften Stift (135) umfasst, die parallel zueinander sind, wobei eines der beiden Enden der ersten Verbindungsstange (150) über den dritten Stift (133) schwenkbar mit dem ersten Greifelement (122) verbunden ist, eines der beiden Enden der zweiten Verbindungsstange (160) über den vierten Stift (134) schwenkbar mit dem zweiten Greifelement (123) verbunden ist, und das andere der beiden Enden der ersten Verbindungsstange (150) und das andere der beiden Enden der Verbindungsstange (160) sind über den fünften Stift (135) schwenkbar mit dem Verbindungselement (124) verbunden.

7. Eingabegerät (100) nach Anspruch 6, wobei der dritte Stift (133) parallel zum ersten Stift (131) ist und/oder eine Mittelachse des dritten Stifts (133) senkrecht

8. Eingabegerät (100) gemäß einem der Ansprüche 1 bis 7, wobei jede der mindestens einen ersten Erfassungskomponente (114) von dem Magnetelement (140) in einer Richtung parallel zur ersten Mittelachse beabstandet ist, und/oder
die mindestens eine erste Erfassungskomponente (114) und das Magnetelement (140) in einer geraden Linie parallel zur ersten Mittelachse angeordnet sind.

9. Eingabegerät (100) nach einem der Ansprüche 1 bis 8, wobei die mindestens eine erste Erfassungskomponente (114) entlang der ersten Mittelachse mit dem Magnetelement (140) ausgerichtet ist.

10. Eingabegerät (100) nach Anspruch 9, wobei zwei zweite Erfassungskomponenten (115) vorgesehen sind,
wobei die beiden zweiten Erfassungskomponenten (115) in einer Richtung parallel zur ersten Mittelachse vom Magnetelement (140) beabstandet sind, und/oder
wobei eine erste Erfassungskomponente (114) vorgesehen ist, die erste Erfassungskomponente (114) und die beiden zweiten Erfassungskomponenten (115) in einer Ebene senkrecht zur ersten Mittelachse angeordnet sind und die beiden zweiten Erfassungskomponenten (115) auf beiden Seiten der ersten Erfassungskomponente (114) angeordnet sind.

11. Eingabegerät (100) nach einem der Ansprüche 1 bis 10, wobei entweder die Montagebasis (110) oder der drehbare Träger (120) oder beide eine Führungsbohrung definieren, sich die Führungsbohrung in einer Richtung parallel zur ersten Mittelachse erstreckt und das Verbindungselement (124) so konfiguriert ist, dass es beweglich durch die Führungsbohrung hindurchgeführt werden kann.

12. Eingabegerät (100) nach Anspruch 11, wobei die Führungsbohrung eine erste Führungsbohrung (116), die durch die Montagebasis (110) definiert ist, und eine zweite Führungsbohrung (125), die durch den drehbaren Träger (120) definiert ist, umfasst, und die erste Führungsöffnung (116) koaxial zur zweiten Führungsöffnung (125) ist.

13. Chirurgischer Roboter, umfassend ein Eingabegerät (100) gemäß einem der Ansprüche 1 bis 12.

## Revendications

1. Dispositif d'entrée (100), comprenant :
une base de montage (110) ;
un support rotatif (120), pouvant tourner par rapport à la base de montage (110) autour d'un premier axe central ;
un mécanisme de préhension (121), disposé sur le support rotatif (120) et pouvant passer d'une position ouverte à une position fermée ;
un élément de connexion (124), relié au mécanisme de préhension (121) et mobile dans une direction parallèle au premier axe central avec l'ouverture et la fermeture du mécanisme de préhension (121), l'élément de connexion pouvant tourner autour du premier axe central avec la rotation support rotatif (120) ;
un élément magnétique (140) ; au moins un premier composant de détection (114), disposé sur la base de montage (110) ; et
au moins un deuxième composant de détection (115), disposé sur la base de montage (110) et configuré pour détecter une intensité de champ magnétique de l'élément magnétique (140) ; et
une carte de traitement de signal (113) montée dans la base de montage (110), la carte de traitement de signal (113) étant configurée pour obtenir un angle inclus entre les éléments du mécanisme de préhension (121) sur la base de l'intensité du champ magnétique de l'élément magnétique (140) détectée par le au moins un deuxième composant de détection (115) ;
**caractérisé en ce que**
l'élément magnétique (140) est disposé sur l'élément de connexion (124), et l'élément magnétique (140) est mobile et rotatif avec l'élément de connexion (124) ; et
le au moins un premier composant de détection (114) est configuré pour détecter les composantes d'un champ magnétique dans les directions x et y dans un plan où se trouve le au moins un premier composant de détection (114) ; et la carte de traitement de signal (113) est en outre configurée pour obtenir un angle de rotation du support rotatif (120) sur la base des composantes du champ magnétique dans les directions x et y détectées par le au moins un premier composant de détection (114).

2. Dispositif d'entrée (100) selon la revendication 1, dans lequel le mécanisme de préhension (121) comprend un premier élément de préhension (122) et un deuxième élément de préhension (123), qui sont respectivement disposés de manière pivotante sur le support rotatif (120).

3. Dispositif d'entrée (100) selon la revendication 2, dans lequel le premier élément de préhension (122) et le deuxième élément de préhension (123) sont disposés respectivement sur deux côtés opposés du support rotatif (120), et le premier élément de préhension (122) et le deuxième élément de préhension (123) sont mobiles de manière coopérative, et/ou
l'élément de connexion (124) comporte une extrémité à laquelle le premier élément de préhension (122) et le deuxième élément de préhension (123) sont reliés de manière pivotante, et une autre extrémité sur laquelle l'élément magnétique (140) est disposé et qui est située à distance du premier élément de préhension (122) et du deuxième élément de préhension (123).

4. Dispositif d'entrée (100) selon la revendication 2 ou la revendication 3, comprenant en outre une première broche (131) et une deuxième broche (132) parallèle à la première broche (131), dans lequel le premier élément de préhension (122) comporte une extrémité reliée de manière pivotante au support rotatif (120) par l'intermédiaire de la première broche (131), et le deuxième élément de préhension (123) a une extrémité reliée de manière pivotante au support rotatif (120) par l'intermédiaire de la deuxième broche (132).

5. Dispositif d'entrée (100) selon la revendication 4, comprenant en outre une première bielle (150) et une deuxième bielle (160), dans lequel la première bielle (150) comporte deux extrémités reliées de manière pivotante au premier élément de préhension (122) et à l'élément de connexion (124), respectivement, et la deuxième bielle (160) comporte deux extrémités reliées de manière pivotante au deuxième élément de préhension (123) et à l'élément de connexion (124), respectivement.

6. Dispositif d'entrée (100) selon la revendication 5, comprenant en outre une troisième broche (133), une quatrième broche (134) et une cinquième broche (135), qui sont parallèles les unes aux autres, dans lequel l'une des deux extrémités de la première bielle (150) est reliée de manière pivotante au premier élément de préhension (122) par l'intermédiaire de la troisième broche (133), l'une des deux extrémités de la deuxième bielle (160) est reliée de manière pivotante au deuxième élément de préhension (123) par l'intermédiaire de la quatrième broche (134), et l'autre des deux extrémités de la première bielle (150) et l'autre des deux extrémités de la bielle (160) sont reliées de manière pivotante à l'élément de connexion (124) par l'intermédiaire de la cinquième broche (135).

7. Dispositif d'entrée (100) selon la revendication 6, dans lequel la troisième broche (133) est parallèle à la première broche (131), et/ou un axe central de la troisième broche (133) est perpendiculaire au premier axe central.

8. Dispositif d'entrée (100) selon l'une quelconque des revendications 1 à 7, dans lequel chacun des au moins un premier composant de détection (114) est espacé de l'élément magnétique (140) dans une direction parallèle au premier axe central, et/ou
le au moins un premier composant de détection (114) et l'élément magnétique (140) sont disposés en ligne droite parallèle au premier axe central.

9. Dispositif d'entrée (100) selon l'une quelconque des revendications 1 à 8, dans lequel le au moins un premier composant de détection (114) est aligné avec l'élément magnétique (140) le long du premier axe central.

10. Dispositif d'entrée (100) selon la revendication 9, dans lequel deux seconds composants de détection (115) sont prévus,
dans lequel les deux seconds composants de détection (115) sont espacés de l'élément magnétique (140) dans une direction parallèle au premier axe central, et/ou
dans lequel un premier composant de détection (114) est prévu, le premier composant de détection (114) et les deux seconds composants de détection (115) sont disposés sur un plan perpendiculaire au premier axe central, et les deux seconds composants de détection (115) sont disposés sur deux côtés du premier composant de détection (114).

11. Dispositif d'entrée (100) selon l'une quelconque des revendications 1 à 10, dans lequel l'une ou les deux parmi la base de montage (110) et le support rotatif (120) définit un trou de guidage, le trou de guidage s'étend dans une direction parallèle au premier axe central, et l'élément de connexion (124) est configuré pour passer de manière mobile à travers le trou de guidage.

12. Dispositif d'entrée (100) selon la revendication 11, dans lequel le trou de guidage comprend un premier trou de guidage (116) défini par la base de montage (110) et un second trou de guidage (125) défini par le support rotatif (120), et le premier trou de guidage (116) est coaxial au second trou de guidage (125).

13. Robot chirurgical, comprenant un dispositif d'entrée (100) selon l'une quelconque des revendications 1 à 12.
